(19) Europäisches Patentamt
European Patent Office
Office européen des brevets

(11) **EP 1 820 519 B1**

(12) **FASCICULE DE BREVET EUROPEEN**

(45) Date de publication et mention
de la délivrance du brevet:
**10.04.2013 Bulletin 2013/15**

(51) Int Cl.:
***A61L 2/28*** *(2006.01)*     ***A61L 2/07*** *(2006.01)*

(21) Numéro de dépôt: **07290114.3**

(22) Date de dépôt: **29.01.2007**

(54) **Procédé et dispositif de contrôle de stérilisation de produits dans un autoclave**

Verfahren und Vorrichtung zur Kontrolle der Sterilisation von Gegenständen in einem Autoklav

Process and device for controlling the sterilization of articles within an autoclave

(84) Etats contractants désignés:
**CH DE FR GB IT LI**

(30) Priorité: **02.02.2006 FR 0600964**

(43) Date de publication de la demande:
**22.08.2007 Bulletin 2007/34**

(73) Titulaire: **MXM**
**06224 Vallauris (FR)**

(72) Inventeurs:
• **Martel, Paul**
**83700 St-Raphael (FR)**
• **Charvin, Guy**
**06600 Antibes (FR)**

(74) Mandataire: **Somnier, Jean-Louis et al**
**Novagraaf Technologies**
**122, rue Edouard Vaillant**
**92593 Levallois Perret Cedex (FR)**

(56) Documents cités:
**WO-A-01/07092         DE-A1- 19 825 166**
**DE-U1- 29 802 948     GB-A- 1 163 917**
**US-A- 3 982 893         US-A- 5 426 428**
**US-A1- 2003 133 830**

**Description**

La présente invention a pour objet un procédé et dispositif de contrôle de stérilisation de produits dans un autoclave.

Le secteur technique de l'invention est le domaine du contrôle des cycles de stérilisation effectués dans les appareils en particulier fermés, de type à pré-vide et gaz stérilisant, afin de vérifier si le cycle de stérilisation s'est effectué dans de bonnes conditions.

La présente invention est particulièrement, mais non exclusivement, destinée à être utilisée dans les appareils à pré-vide de stérilisation par la vapeur d'eau tels que les autoclaves assurant la stérilisation d'équipements médicaux et d'hôpitaux et pour lesquels il est d'autant plus nécessaire de savoir s'ils ont été efficacement et correctement stérilisés, tels que par exemple par une exposition à la vapeur d'eau à 134°C pendant 18 minutes. Et ces conditions doivent être réalisées même à l'intérieur des contenants dans lesquels sont placés lesdits équipements et qui sont destinés à préserver l'état stérile de ceux-ci jusqu'à leur utilisation après leur sortie de l'autoclave.

Ces contenants sont donc conçus pour, d'une part empêcher la pénétration de tout fluide ou autre polluant lors de leur stockage et leur transport jusqu'au lieu de leur ouverture pour usage des produits qu'ils contiennent et, d'autre part, être suffisamment non étanches pour permettre une mise sous vide et l'entrée du fluide, telle que la vapeur, de stérilisation grâce aux différentiels de pression créés par la mise sous vide préalable du contenant lors du cycle de stérilisation dans l'autoclave : ceci est obtenu soit par des systèmes à soupapes qui s'ouvrent dans un sens et dans l'autre quand la pression externe au contenant est suffisamment différente de la pression interne pour permettre dans un sens le vide et dans l'autre sens de laisser entrer le fluide de stérilisation tel que la vapeur, soit par une réduction des ouvertures de communication entre leur volume intérieur et l'extérieur, et une protection de ces ouvertures tant mécanique que par divers filtres tels qu'en papier qui les obturent ; ces divers éléments permettent de qualifier le volume intérieur de ces contenants de « confiné » en complément de la non-étanchéité.

On définira à titre d'exemple cette caractéristique de « non étanchéité » et de confinement par une valeur de surface de section de passage autorisée par ces filtres par rapport au volume interne des contenants égal à au moins 0,1% $M^{-1}$, et de préférence 0,5% $M^{-1}$ et plus éventuellement, mais tout en restant très faible, afin de permettre la mise sous vide, puis l'entrée du fluide de vapeur de stérilisation dans un temps raisonnable compatible avec la durée totale d'un cycle de stérilisation.

A ce jour, un tel contrôle de la « bonne » stérilisation des produits contenus dans un contenant (c'est-à-dire que par exemple leur température a été maintenue entre 121 et 134°C pendant 3 à 25 minutes, et/ou que la variation de pression en fonction de la température a bien suivi la courbe d'équilibre de vapeur saturante, dite courbe de Regnault) ne peut être effectué qu'au moment de l'ouverture du contenant pour en utiliser les équipements placés à l'intérieur et censés avoir été bien stérilisés : on place pour cela avant leur fermeture et leur introduction dans l'autoclave, à l'intérieur de chaque contenant soit un équipement assez important embarqué capable de mesurer et d'enregistrer en continu la température et la pression, soit un indicateur par encre physicochimique réactive à la vapeur, et que l'on consulte au moment de l'ouverture.

Un tel contrôle est cependant décalé un long moment après la stérilisation, alors que le contenant a pu être déplacé jusqu'à son lieu d'utilisation qui peut être éloigné de l'autoclave : en cas alors de non-conformité, les équipements ne sont pas utilisables et il faut alors les renvoyer à stériliser, ce qui crée une perte de temps et nécessite de disposer d'une avance de stock important d'équipements ayant suivi un cycle de stérilisation et en attente d'être utilisés pour pallier au risque de mauvaise stérilisation de certains.

Le problème posé est donc de pouvoir contrôler celle-ci au plus tôt, soit avant soit juste après la sortie des contenants de l'autoclave, même si c'est bien avant d'avoir à utiliser les équipements qui contiennent, tout en préservant ensuite la stérilisation de ceux-ci jusqu'à leur utilisation, sans nécessiter la mise en oeuvre d'un matériel et/ou d'un procédé compliqué, lourd à mettre en oeuvre et/ou coûteux, et ce contrôle doit pouvoir se faire avec tout contenant existant non étanche et confiné, en particulier ceux entièrement métalliques sans adaptation particulière comme une fenêtre transparente telle que décrite dans le modèle d'utilité allemand DE 29802948.

Une solution au problème posé est un procédé de contrôle de stérilisation de produits dans un autoclave, lesquels produits étant placés dans au moins un contenant fermé, non étanche et confiné, , réalisé en métal et sans fenêtre transparente, de tout type adapté à la stérilisation et apte à préserver l'état stérile des produits jusqu'à leur utilisation après leur sortie de l'autoclave, et suivant lequel on dispose dans ce même contenant un dispositif embarqué et autonome pour la mesure dans le dit contenant d'au moins un paramètre de stérilisation et tel qu'on enregistre dans le dit dispositif embarqué, pendant au moins une partie de la durée du cycle de stérilisation et suivant une périodicité prédéfinie, au moins une valeur représentative de la mesure du dit au moins paramètre.

Suivant l'invention on mesure au moins un paramètre de stérilisation parmi la pression, l'humidité, et la température, et on ne transmet au moins une de ces valeurs représentatives, directement par le dispositif embarqué et au travers du contenant resté fermé, qu'à la fin du cycle de stérilisation, en utilisant une transmission sans fil par rayonnement électromagnétique d'ondes de fréquence inférieures à 250 000 GHz, de préférence en dessous de 30 000 GHz, et même de 1 000 GHz, et pouvant aller jusqu'aux grandes ondes hertziennes de 3 KHz en passant par toutes les ondes hertziennes dites radioélectriques comprise entre 9 KHz et 3 000 GHz, telles que les microondes, les UHF, VHF, HF, MF, LF et VLF, ou par induction magnétique.

Une autre solution au problème posé est un dispositif de contrôle embarqué et un contenant dans lequel est placé ce dispositif de contrôle de stérilisation de produits dans un autoclave, lequel contenant fermé dans lequel sont placés les dits produits, est non étanche et confiné, , réalisé en métal et sans fenêtre transparente, et de tout type apte à préserver l'état stérile des produits jusqu'à leur utilisation après leur sortie de l'autoclave, lequel dispositif étant autonome, disposant de batteries électriques, et d'un circuit de composants électroniques, et placé dans ledit contenant pour y mesurer au moins un paramètre de stérilisation et tel qu'au moins un desdits composants électroniques est programmé pour exécuter le procédé ci-dessus et l'enveloppe externe du dit dispositif est perméable à un rayonnement électromagnétique ou induction magnétique sans fil.

Sachant que de nombreux contenants sont réalisés en métal tels que ceux fabriqués par la société WAGNER GmbH et commercialisés sous la marque STERISET®, et du fait de leur caractéristique de non étanchéité très faible, donnant une continuité de parois à l'enveloppe de ces contenants, un homme du métier considérait une telle transmission au travers de celui-ci comme impossible du fait de l'effet de cage de FARADAY: l'inventeur de la présente invention a donc vaincu ce préjugé et a mis au point divers procédés tels que décrits ci-après pour simplifier au maximum l'acquisition des données et de n'avoir à transmettre qu'un minimum d'information, réduisant ainsi la taille, les capacités et les batteries de fonctionnement du dispositif apte à mettre en oeuvre de tels procédés, simplifiant sa mise en oeuvre et réduisant le coût de fabrication.

En particulier, un tel dispositif n'est donc pas uniquement un appareil d'enregistrement dans le sens où il n'enregistre pas et ne conserve pas toutes les données relevées au fur et à mesure du cycle de stérilisation : celles-ci sont traitées localement dans le dispositif, puis perdues et c'est uniquement le ou les résultats de ce traitement qui est enregistré et transmis à la fin du cycle de stérilisation et à la demande vers un lecteur externe tel que défini ci-après.

Grâce au procédé de l'invention tel que présenté ci-dessus et décrit plus précisément ci-après, on peut lire avec un lecteur externe apte à recevoir les ondes hertziennes ou à capter l'induction magnétique émises par le dispositif selon l'invention, et sans que le contenant dispose d'une adaptation particulière telle qu'une fenêtre transparente ou une antenne extérieure, et sans avoir à ouvrir les contenants, et cela avant ou dès leur sortie de l'autoclave, les informations stockées dans lesdits dispositifs suivant l'invention, disposés à l'intérieur préalablement de ceux-ci : le lecteur externe permet alors à l'opérateur de vérifier, par contrôle des valeurs représentatives transmises de la mesure du ou des paramètres mesurés et enregistrés, si le cycle de stérilisation s'est bien déroulé à l'intérieur du contenant, suivant des critères préétablis ; et si ceux-ci ne sont pas remplis, il peut alors immédiatement vérifier s'il n'y a pas un problème sur le contenant (tel que les filtres bouchés ou autres) et remettre celui-ci dans l'autoclave pour un nouveau cycle de stérilisation.

Seuls les contenants ayant correctement suivi un cycle de stérilisation conforme sont alors adressés aux utilisateurs potentiels, qui pourront les ouvrir au moment voulu, en étant sûrs qu'ils trouveront des équipements correctement stérilisés.

Le résultat est de nouveaux procédés et dispositifs de contrôle de stérilisation de produits dans un autoclave répondant au problème posé de contrôle de la qualité de la stérilisation, au plus tôt soit avant soit après la sortie des contenants de l'autoclave, tout en préservant la stérilisation des produits et équipements situés dans les contenants, puisqu'il n'y a plus besoin d'ouvrir ceux-ci pour un tel contrôle, et cela avec un dispositif simple et peu coûteux, comme décrit plus précisément ci-après à titre d'exemple, et en utilisant tout type de contenant existant non étanche et confiné, en particulier métallique et/ou à parois opaques.

On pourrait citer d''autres avantages de la présente invention, mais ceux cités ci-dessus en montrent déjà suffisamment pour en prouver la nouveauté et l'intérêt. La description et les dessins ci-joints représentent un exemple de réalisation de l'invention, mais n'ont aucun caractère limitatif : d'autres réalisations sont possibles dans le cadre de la portée et de l'étendue de cette invention.

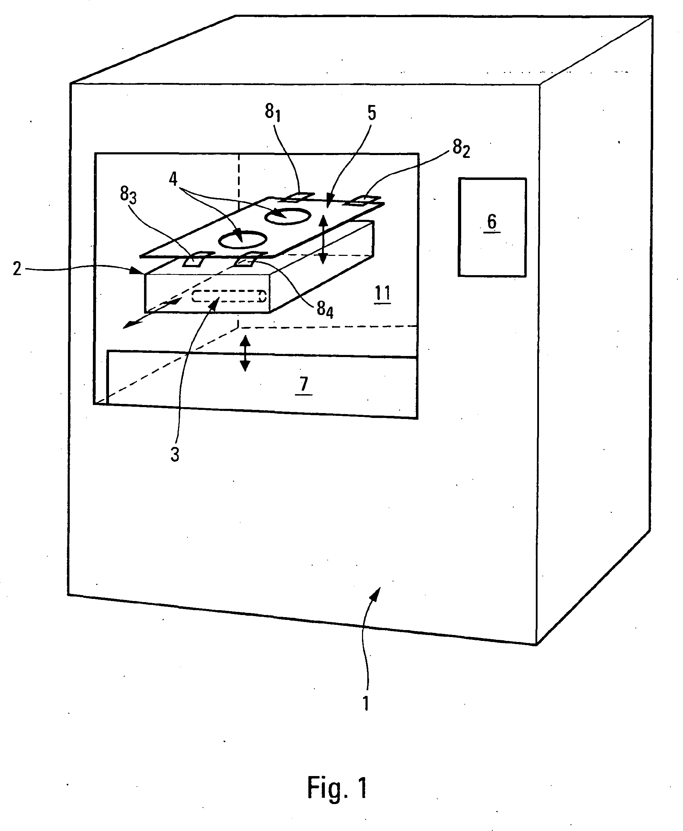

La figure 1 est une vue en perspective d'ensemble d'un contenant pouvant être rempli de produits ou équipements à stériliser et d'un dispositif suivant l'invention, prêt à être fermé et être introduit dans un autoclave pour subir un cycle de stérilisation.

La figure 2 est un exemple de schéma électronique et électrique d'un circuit de composants constituant un dispositif suivant l'invention.

On place dans au moins un contenant fermé 2, non étanche et confiné, réalisé en métal, de tout type adapté à la stérilisation, des produits que l'on veut stériliser et un dispositif embarqué 3 et autonome pour la mesure 12 dans ledit contenant 2 d'au moins un paramètre de stérilisation.

Ledit contenant comporte au moins une partie de paroi non étanche 4, mais non transparente, qui est généralement portée par le couvercle 5 et qui est composée par des filtres de type papier ayant les caractéristiques déjà définies précédemment.

On solidarise et verrouille ledit couvercle 5 sur la base du contenant par divers systèmes de fermeture 8. On dispose alors ledit contenant dans la chambre de stérilisation 11 d'un autoclave 1 et on peut ainsi disposer côté à côté et les uns sur les autres divers autres contenants, enfermant chacun des équipements ou produits à stériliser ainsi qu'un dispositif embarqué 3 selon l'invention. On peut alors fermer la porte d'accès et de fermeture 7 de l'autoclave 1 et commander le démarrage du cycle de stérilisation par un pupitre de commande 6.

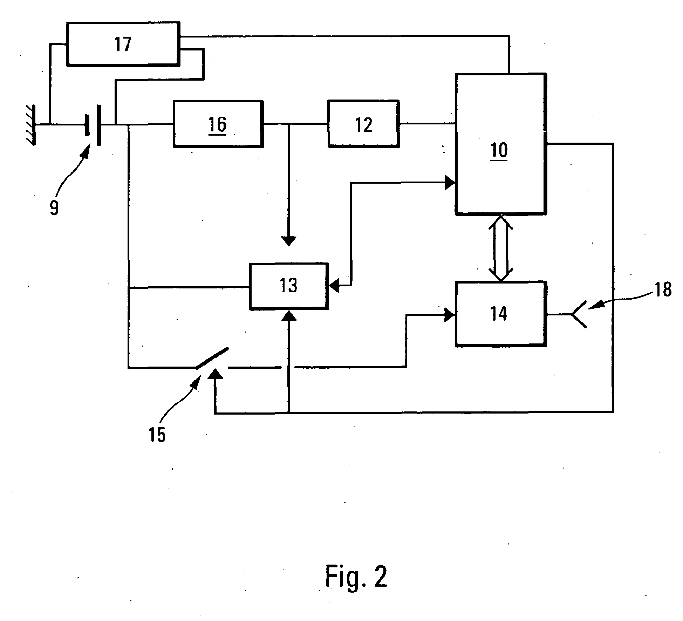

Grâce au dispositif embarqué 3, représenté sur la figure 2, on enregistre dans une mémoire 13 pendant au moins une partie de la durée du cycle de stérilisation et suivant une période prédéfinie, au moins une valeur représentative de la mesure dudit au moins paramètre relevé grâce à un capteur 12.

On ne transmet ensuite grâce à un émetteur 14 au moins une de ces valeurs représentatives au travers du contenant resté fermé 2 soit quand celui-ci est encore dans l'autoclave 1 et à tout moment du cycle de stérilisation, soit directement après l'extraction du contenant 2 de l'intérieur de l'autoclave 1, soit un peu plus tard. De préférence on ne transmet la au moins valeur représentative qu'à la fin du cycle de stérilisation et on en déduit la façon dont ce cycle de stérilisation s'est effectuée.

Pour cela, ladite valeur représentative de la mesure est transmise à un lecteur externe non représenté sur les figures, apte à relever des valeurs représentatives des mesures de plusieurs dispositifs 3 placés simultanément chacun dans un contenant 2 différent; ainsi chacun des contenants placés tous ensemble dans un autoclave 1 pour suivre un même cycle de stérilisation comprennent chacun un dispositif 3 dont le contrôle des mesures relevées peut être effectué à la fin du cycle de stérilisation par un même lecteur.

Pendant le cycle de stérilisation, au cours duquel sont effectués et enregistrés la mesure du ou des paramètres, l'émission 14 peut être désactivée grâce à un interrupteur 15 qui ne se ferme que quand la température dans le contenant redescend au-dessous d'une certaine température telle que la température ambiante, signifiant que le cycle de stérilisation est terminé et que le contenant a été sorti de l'autoclave.

En sens inverse, un autre interrupteur 16 de mise en marche n'est fermé que quand la température mesurée à l'intérieur du contenant a dépassé un seuil minimum, de 100°C par exemple ; un tel interrupteur 16 peut être constitué d'un bilame.

Le dispositif 3 est autonome, disposant de batteries électriques 9 et d'un circuit de composants électroniques, dont au moins un de ces composants 10 est programmé pour exécuter le procédé selon la présente invention, lequel dispositif est apte à être placé dans ledit contenant 2 pour y mesurer au moins un paramètre de stérilisation.

L'enveloppe externe du dispositif est perméable à un rayonnement électromagnétique ou induction magnétique.

Le circuit de composants électroniques comporte un convertisseur analogique-numérique 10 placé le plus en avant possible près du capteur 12 apte à mesurer le au moins paramètre de stérilisation, ledit circuit de composants électroniques étant également apte à auto-étalonner ce capteur de mesure 12.

Le dispositif 1 comporte également un testeur 17 de batterie 9 et utilise une transmission sans fil 18 par rayonnement électromagnétique, d'ondes de fréquence inférieures à 250 000 GHz, de préférence en dessous de 30 000 GHz, et même de 1 000 GHz, et pouvant aller jusqu'aux grandes ondes hertziennes de 3 KHz en passant par toutes les ondes hertziennes dites radioélectriques comprises entre 9 KHz et 3 000 GHz, telles que les microondes, les UHF, VHF, HF, MF, LF et VLF,_et de préférence courantes telles que 800 Mhz ou 2,4 Ghz, ou par induction magnétique.

Dans un mode particulier de réalisation on peut également utiliser la variation de température à l'intérieur du contenant 2 pour créer un courant électrique apte à recharger la batterie 9 de ce dispositif, mais cette option n'est pas représentée sur la figure 2.

Pour minimiser la taille de la mémoire 13 et n'avoir à transmettre qu'un minimum d'information en fin de cycle, au fur et à mesure des relevés de la mesure 12 dudit au moins paramètre dans le dispositif embarqué 3, en tenant compte au moins d'une des valeurs de mesure et de celles précédemment enregistrées, on effectue tout calcul 10 prédéfini, tel que l'exemple donné ci-après, permettant d'obtenir une nouvelle valeur représentative de ladite mesure et on enregistre 13 cette dernière valeur ainsi calculée à la place de la précédente, permettant l'effacement du ou des valeurs précédemment enregistrées.

Dans un mode particulier de réalisation, le procédé de l'invention est tel que :

- on compare 10 dans ledit dispositif embarqué pendant la durée du cycle de stérilisation, la valeur d'au moins un paramètre mesurée avec au moins une valeur prédéterminée,
- quand au moins cette dernière valeur est atteinte, on relève cette information comme valide et constituant la valeur représentative de la mesure dudit au moins paramètre,
- on transmet 14 alors ladite information soit avant soit après l'extraction du contenant 2 de l'intérieur de l'autoclave 1 et à travers le contenant 2 resté fermé, et
- on en déduit que le cycle de stérilisation s'est bien effectué dans des conditions minimales satisfaisantes.

Dans un mode préférentiel de réalisation, la comparaison des valeurs et l'enregistrement de l'information valide et représentative de cette mesure étant réalisée dans le dispositif embarqué 3, on relève et transmet ladite information sous une forme binaire.

La valeur prédéterminée du paramètre mesuré est par exemple un seuil tel que de pression, d'humidité ou de température et on relève l'information valide quand d'une part la valeur dudit paramètre mesuré par le capteur 12, (sachant qu'il peut y en avoir plusieurs si l'on veut mesurer plusieurs de ces paramètres : pression, humidité, température) a atteint ce seuil et d'autre part qu'après un temps donné de maintien de la valeur de ce paramètre au-dessus de ce seuil.

Dans un autre mode de réalisation, on considère la courbe de Regnault comme un ensemble de valeurs prédéterminées de la température à contrôler dans l'enceinte en fonction de la pression, on compare 10 pendant la durée du cycle de stérilisation les valeurs mesurées de la température et de la pression dans le contenant 3 avec les valeurs de ladite courbe, et on relève 13 l'information de validation quand ces valeurs mesurées correspondent à celles de la courbe de Regnault pendant un temps donné.

Par ailleurs, comme en général le au moins paramètre mesuré dans ledit contenant peut être la température, on calcule à tout instant la force stérilisatrice $F_0$ telle que $F_0 = \int_0^t temp*dt$ et on transmet la valeur obtenue sous la forme d'un chiffre.

Grâce à la simplicité du procédé suivant l'invention et du schéma électronique du dispositif représenté sur la figure 2, celui-ci peut être miniaturisé et glisser dans une enveloppe externe de forme cylindrique dont le diamètre peut être de 3 cm et d'une longueur de 12 à 15 cm, de type tuyau en plastique fermé à ses deux extrémités.

## Revendications

**1.** Procédé de contrôle de stérilisation de produits dans un autoclave (1), lesquels produits étant placés dans au moins un contenant fermé (2), non étanche et confiné, réalisé en métal et sans fenêtre transparente, de tout type adapté à la stérilisation et apte à préserver l'état stérile des produits jusqu'à leur utilisation après leur sortie de l'autoclave, et suivant lequel on dispose dans ce même contenant un dispositif embarqué (3) et autonome pour la mesure (12) dans le dit contenant (2) d'au moins un paramètre de stérilisation et tel qu'on enregistre (13) dans ledit dispositif embarqué (3), pendant au moins une partie de la durée du cycle de stérilisation et suivant une périodicité prédéfinie, au moins une valeur représentative de la mesure du dit au moins paramètre, dans lequel :

- on mesure au moins un paramètre de stérilisation parmi la pression, l'humidité, et la température,
- on ne transmet (14) au moins une de ces valeurs représentatives, directement au travers du contenant resté fermé (2) qu'à la fin du cycle de stérilisation,
- on utilise pour cela une transmission sans fil (18) par rayonnement électromagnétique d'ondes de fréquence inférieures à 250 000 GHZ ou par induction magnétique.

**2.** Procédé selon la revendication 1 , **caractérisé en ce que** le au moins paramètre mesuré dans ledit contenant étant la température, on calcule à tout instant la force stérilisatrice $F_0$ telle que

$$F_0 = \int_0^t temp*dt$$ et on transmet la valeur obtenue sous la forme d'un chiffre

**3.** Procédé suivant l'une quelconque des revendication 1 et 2 **caractérisé en ce que**

- on compare (10), dans ledit dispositif embarqué et pendant la durée du cycle de stérilisation, la valeur d'au moins un paramètre mesuré avec au moins une valeur prédéterminée,
- quand au moins cette dernière valeur est atteinte, on relève cette information comme valide et constituant la valeur représentative de la mesure du dit au moins paramètre
- on transmet (14) alors la dite information après l'extraction du contenant (2) de l'intérieur de l'autoclave (1) et à travers le contenant (2) resté fermé, et
- on en déduit que le cycle de stérilisation s'est bien effectué dans des conditions minimales satisfaisantes.

**4.** Procédé selon la revendication 3 **caractérisé en ce que** la mesure de paramètre, la comparaison des valeurs et l'enregistrement de l'information valide et représentative de cette mesure étant réalisés dans le dispositif embarqué (3), on relève et transmet la dite information sous une forme binaire.

**5.** Procédé selon l'une quelconque des revendications 3 ou 4 **caractérisé en ce que** la valeur prédéterminée du paramètre mesuré est un seuil, et on relève l'information valide quand d'une part la valeur du dit paramètre a atteint ce seuil et d'autre part qu'après un temps donné de maintien de la valeur de ce paramètre au dessus de ce seuil.

**6.** Procédé selon l'une quelconque des revendications 3 à 5 **caractérisé en ce que** on considère la courbe de Regnault comme un ensemble de valeurs prédéterminés de la température à contrôler dans l'enceinte en fonction de la pression, on compare (10) pendant la durée du cycle de stérilisation les valeurs mesurées de la température et de la pression dans le contenant (3) avec les valeurs de la dite courbe, et on relève (13) l'information de validation quand ces valeurs mesurées correspondent à celles de la courbe de Regnault pendant un temps donné.

**7.** Procédé selon l'une quelconque des revendications 1 à 6 **caractérisé en ce que** l'on transmet la dite valeur représentative de la mesure à un lecteur externe apte à relever les valeurs représentatives des mesures de plusieurs dispositifs placés simultanément chacun dans un contenant.

**8.** Procédé selon l'une quelconque des revendications 1 à 7 **caractérisé en ce que** l'on utilise la variation de température à l'intérieur du contenant pour créer un courant électrique apte à recharger la batterie (9) du dispositif.

**9.** Procédé selon l'une quelconque des revendications 1 à 8 , **caractérisé en ce que** l'on utilise pour réaliser la transmission sans fil (18) par rayonnement électromagnétique, les ondes hertziennes de fréquences courantes telles que 800 Mhz ou 2,4 Ghz.

**10.** Dispositif de contrôle embarqué et contenant dans lequel est placé ce dispositif de contrôle de stérilisation de produits dans un autoclave (1), lequel contenant fermé (2) dans lequel sont placés les dits produits, est non étanche et confiné, , réalisé en métal et sans fenêtre transparente, et de tout type apte à préserver l'état stérile des produits jusqu'à leur utilisation après leur sortie de l'autoclave, lequel dispositif étant autonome, disposant de batteries électriques (9), et d'un circuit de composants électroniques, et placé dans ledit contenant (2) pour y mesurer au moins un paramètre de stérilisation, au moins un desdits composants électroniques (10) étant programmé pour exécuter le procédé selon l'une quelconque des revendications 1 à 9 et l'enveloppe externe du dit dispositif étant perméable à un rayonnement électromagnétique ou induction magnétique sans fil.

**11.** Dispositif et contenant selon la revendication 10 **caractérisé en ce que** le circuit de composants électroniques comporte un convertisseur analogique-numérique (10) placé le plus en avant possible prés du capteur (12) apte à mesurer le au moins paramètre de stérilisation.

**12.** Dispositif et contenant selon l'une quelconque des revendications 10 à 11 **caractérisé en ce que** ledit circuit de composants électroniques est apte à auto-étalonner le capteur de mesure (12) du au moins paramètre de stérilisation.

**Claims**

**1.** Method for monitoring the sterilization of products in an autoclave (1), said products being placed in at least one closed container (2), not seal-tight and confined, made of metal and with no transparent window, of any type suited to the sterilization and capable of preserving the sterile state of the products until their use after they have left the autoclave, and according to which there is, in this same container, a standalone and embarked device (3) for measuring (12), in said container (2), at least one sterilization parameter, and such that there is recorded (13) in said embarked device (3), during at least a part of the duration of the sterilization cycle and according to a predefined periodicity, at least one value representative of the measurement of said at least one parameter, whereby:

- at least one sterilization parameter among the pressure, humidity and temperature is measured,
- at least one of these representative values is transmitted (14), directly through the still closed container (2), only at the end of the sterilization cycle,
- for this, a wireless transmission (18) is used based on electromagnetic radiation of waves of frequency less than 250 000 GHZ or by magnetic induction.

**2.** Method according to Claim 1, **characterized in that**, the at least one parameter measured in said container being the temperature, the sterilizing force $F_0$ such that $F_0 = \int_0^1 temp * dt$ is calculated at any instant and the value obtained is transmitted in the form of a digit.

**3.** Method according to any one of Claims 1 and 2, **characterized in that**

- the value of at least one measured parameter is compared (10), in said embarked device and for the duration of the sterilization cycle, with at least one predetermined value,
- when at least this latter value is reached, this information is recorded as valid and constitutes the value representative of the measurement of said at least one parameter,
- said information is then transmitted (14) after the extraction of the container (2) from the interior of the autoclave (1) and through the still closed container (2), and
- it is deduced therefrom that the sterilization cycle has been correctly completed in satisfactory minimal conditions.

**4.** Method according to Claim 3, **characterized in that** the parameter measurement, the comparison of the values and the recording of the valid information representative of this measurement being performed in the embarked device (3), said information is recorded and transmitted in a binary form.

**5.** Method according to any one of Claims 3 or 4, **characterized in that** the predetermined value of the measured parameter is a threshold, and the valid information is recorded when, on the one hand, the value of said parameter has reached this threshold and, on the other hand, only after a given time maintaining the value of this parameter above this threshold.

6. Method according to any one of Claims 3 to 5, **characterized in that** the Regnault curve is considered as a set of predetermined values of the temperature to be monitored in the chamber as a function of the pressure, the measured values of the temperature and of the pressure in the container (2) are compared (10), for the duration of the sterilization cycle, with the values of the said curve, and the validation information is recorded (13) when these measured values correspond to those of the Regnault curve for a given time.

7. Method according to any one of Claims 1 to 6, **characterized in that** the said value representative of the measurement is sent to an external reader capable of recording the values representative of the measurements of a plurality of devices, each placed simultaneously in a container.

8. Method according to any one of Claims 1 to 7, **characterized in that** the temperature variation inside the container is used to create an electrical current capable of recharging the battery (9) of the device.

9. Method according to any one of Claims 1 to 8, **characterized in that** the microwaves of current frequencies such as 800 Mhz or 2.4 Ghz are used to perform the wireless transmission (18).

10. Embedded monitoring device and container in which is placed this device for monitoring the sterilization of products in an autoclave (1), said closed container (2) in which are placed said products is not seal-tight and contained, made of metal and with no transparent window, and of any type capable of preserving the sterile state of the products until their use after they leave the autoclave, said device being standalone, having electric batteries (9), and a circuit of electronic components, and placed in said container (2) to measure therein at least one sterilization parameter, at least one of said electronic components (10) being programmed to execute the method according to any one of Claims 1 to 9 and the outer jacket of said device being permeable to a wireless electromagnetic radiation or magnetic induction.

11. Device and container according to Claim 10, **characterized in that** the circuit of electronic components comprises an analogue-digital converter (10) placed as far forward as possible close to the sensor (12) capable of measuring the at least one sterilization parameter.

12. Device and container according to any one of Claims 10 to 11, **characterized in that** said circuit of electronic components is capable of self-calibrating the sensor (12) for measuring the at least one sterilization parameter.

## Patentansprüche

1. Verfahren zum Steuern der Sterilisation von Produkten in einem Autoklaven (1), wobei die Produkte in wenigstens einem nicht dichten und abgekapselten, geschlossenen Behälter (2), der aus Metall und ohne lichtdurchlässiges Fenster verwirklicht ist und von irgendeinem Typ ist, der für die Sterilisation geeignet ist und den sterilen Zustand der Produkte bis zu ihrer Verwendung nach dem Verlassen des Autoklaven aufrechterhalten kann, angeordnet sind, wobei in diesem Behälter eine bordinterne und autonome Vorrichtung (3), um in dem Behälter (2) wenigstens einen Sterilisationsparameter zu messen (12), enthält, derart, dass in der bordinternen Vorrichtung (3) wenigstens während eines Teils der Dauer des Sterilisationszyklus und mit einer im Voraus definierten Periodizität wenigstens ein Wert aufgezeichnet wird (13), der die Messung des wenigstens einen Parameters repräsentiert, wobei

   - wenigstens ein Sterilisationsparameter, der aus dem Druck, der Feuchtigkeit und der Temperatur gewählt ist, gemessen wird,
   - wenigstens einer dieser repräsentativen Werte direkt durch den geschlossen bleibenden Behälter (2) nur am Ende des Sterilisationszyklus gesendet wird (14),
   - hierzu eine drahtlose Übertragung (18) durch elektromagnetische Strahlung mit Wellen mit einer Frequenz kleiner als 250 000 GHz oder durch magnetische Induktion verwendet wird.

2. Verfahren nach Anspruch 1, **dadurch gekennzeichnet, dass** dann, wenn der wenigstens eine gemessene Parameter in dem Behälter die Temperatur ist, zu jedem Zeitpunkt die sterilisierende Kraft $F_0$ berechnet wird, derart, dass

$$F_0 = \int_0^1 temp * dt,$$

wobei der erhaltene Wert in Form einer Zahl gesendet wird.

3. Verfahren nach einem der Ansprüche 1 und 2, **dadurch gekennzeichnet, dass**

   - in der bordinternen Vorrichtung während der Dauer des Sterilisationszyklus der Wert wenigstens eines gemessenen Parameters mit wenigstens einem vorgegebenen Wert verglichen wird (10),

- dann, wenn wenigstens dieser letztere Wert erhalten wird, diese Informationen als gültig und so abgegriffen werden, dass sie den die Messung des wenigstens einen Parameters repräsentierenden Wert bilden,

- anschließend diese Informationen nach der Entnahme des Behälters (2) aus dem Innenraum des Autoklaven (1) und durch den geschlossen bleibenden Behälter (2) gesendet werden (14) und

- daraus abgeleitet wird, dass der Sterilisationszyklus unter zufriedenstellenden Minimalbedingungen erfolgreich erfolgt ist.

4. Verfahren nach Anspruch 3, **dadurch gekennzeichnet, dass** dann, wenn die Messung des Parameters, der Vergleich der Werte und die Aufzeichnung der Informationen, die gültig sind und diese Messung repräsentieren, in der bordinternen Vorrichtung (3) ausgeführt werden, diese Informationen abgegriffen und in binärer Form gesendet werden.

5. Verfahren nach einem der Ansprüche 3 oder 4, **dadurch gekennzeichnet, dass** der vorgegebene Wert des gemessenen Parameters ein Schwellenwert ist und dass die gültigen Informationen abgegriffen werden, wenn einerseits der Wert des Parameters diesen Schwellenwert erreicht hat und wenn andererseits der Wert dieses Parameters für eine gegebene Zeit oberhalb dieses Schwellenwertes gehalten worden ist.

6. Verfahren nach einem der Ansprüche 3 bis 5, **dadurch gekennzeichnet, dass** die Regnault-Kurve als eine Gesamtheit vorgegebener Werte der zu steuernden Temperatur in der Umschließung als Funktion des Drucks angesehen wird, während der Dauer des Sterilisationszyklus die gemessenen Werte der Temperatur und des Drucks in dem Behälter (3) mit den Werten der Kurve verglichen werden (10) und die Validierungsinformationen abgegriffen werden (13), wenn diese gemessenen Werte jenen der Regnault-Kurve während einer gegebenen Zeit entsprechen.

7. Verfahren nach einem der Ansprüche 1 bis 6, **dadurch gekennzeichnet, dass** der genannte Wert, der die Messung repräsentiert, an eine externe Leseeinrichtung gesendet wird, die diese Werte, die die Messungen von mehreren Vorrichtungen repräsentieren, die gleichzeitig in jeweils einem Behälter angeordnet sind, abgreifen kann.

8. Verfahren nach einem der Ansprüche 1 bis 7, **dadurch gekennzeichnet, dass** die Veränderung der Temperatur im Innenraum des Behälters verwendet wird, um einen elektrischen Strom zu erzeugen, der die Batterie (9) der Vorrichtung wieder aufladen kann.

9. Verfahren nach einem der Ansprüche 1 bis 8, **dadurch gekennzeichnet, dass** für die Ausführung der drahtlosen Übertragung (18) durch elektromagnetische Strahlung Hertzsche Wellen mit regulären Frequenzen von 800 MHz oder 2,4 GHz verwendet werden.

10. Bordinterne Steuervorrichtung und Behälter, in dem diese Vorrichtung für die Steuerung der Sterilisation von Produkten in einem Autoklaven (1) angeordnet ist, wobei der geschlossene Behälter (2), in dem die Produkte angeordnet sind, nicht dicht und abgekapselt ist, aus Metall und ohne lichtdurchlässiges Fenster verwirklicht ist und von irgendeinem Typ ist, der den sterilen Zustand der Produkte aufrechterhalten kann, bis sie nach ihrer Entnahme aus dem Autoklaven verwendet werden, wobei die Vorrichtung autonom ist, elektrische Batterien (9) und eine Schaltung aus elektronischen Komponenten enthält und in dem Behälter (2) angeordnet ist, um darin wenigstens einen Sterilisationsparameter zu messen, wobei wenigstens eine der elektronischen Komponenten (10) programmiert ist, um das Verfahren nach einem der Ansprüche 1 bis 9 auszuführen, und die äußere Hülle der Vorrichtung für drahtlose elektromagnetische Strahlung oder magnetische Induktion durchlässig ist.

11. Vorrichtung und Behälter nach Anspruch 10, **dadurch gekennzeichnet, dass** die Schaltung aus elektronischen Komponenten einen Analog/Digital-Umsetzer (10) umfasst, der so nahe wie möglich bei dem Sensor (12) angeordnet ist und den wenigstens einen Sterilisationsparameter messen kann.

12. Vorrichtung und Behälter nach einem der Ansprüche 10 bis 11, **dadurch gekennzeichnet, dass** die Schaltung aus elektronischen Komponenten den Sensor (12) zum Messen des wenigstens einen Sterilisationsparameters selbst kalibrieren kann.

Fig. 1

Fig. 2

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- DE 29802948